# EUROPÄISCHE PATENTANMELDUNG

(11) **EP 4 249 028 A2**
(43) Veröffentlichungstag der Anmeldung: **27.09.2023**
(21) Anmeldenummer: 23020317.6
(22) Anmeldetag: 14.04.2020
(51) Int. Cl.: A61M 16/16

(54) **SYSTEM ZUR ATEMGASVERSORGUNG**

(30) Priorität: 24.04.2019 DE 102019110631
(62) Teilanmeldung aus: 20000150.1
(71) Anmelder: Löwenstein Medical Technology S.A., 2557 Luxembourg (LU)
(72) Erfinder: SCHWAIBOLD, Matthias, 76228 Karlsruhe (DE); TIEMANN, Björn, 22359 Hamburg (DE); GÖBEL, Christof, 22457 Hamburg (DE); DÖMER, Benno, 76275 Ettlingen (DE); ADAMETZ, Bemjamin, 22609 Hamburg (DE)
(74) Vertreter: Marx, Thomas

(57) **Zusammenfassung**

Die vorliegende Erfindung betrifft ein System und Verfahren zur Atemgasversorgung mit einer Beatmungseinrichtung (2) mit einer Atemgasquelle (3) zur Erzeugung einer Atemgasströmung und mit einer Steuereinrichtung (4). Die Steuereinrichtung (4) ist dazu geeignet und ausgebildet, mittels der Beatmungseinrichtung (2) einen definierten Atemgasfluss für eine Beatmung zu erzeugen. Dabei ist die Steuereinrichtung (4) dazu geeignet und ausgebildet ist, mittels einer Sensoreinrichtung (5) einen für einen Druck charakteristischen Parameter zu überwachen. Der Parameter charakterisiert denjenigen Druck, mit welchem die Beatmungseinrichtung (2) die Atemgasströmung zur Aufrechterhaltung des definierten Atemgasflusses beaufschlagt, um eine Atemtätigkeit des Patienten auszugleichen. Die Steuereinrichtung (4) ist dazu geeignet und ausgebildet ist, einen Verlauf des Parameters über die Zeit als einen Druckverlauf zu registrieren.

## Beschreibung

Die vorliegende Erfindung betrifft ein System zur Atemgasversorgung und ein Verfahren zum Betreiben eines solchen Systems. Das System umfasst wenigstens eine Beatmungseinrichtung mit wenigstens einer Atemgasquelle zur Erzeugung einer Atemgasströmung und wenigstens eine Steuereinrichtung. Die Steuereinrichtung erzeugt mittels der Beatmungseinrichtung einen definierten Atemgasfluss für eine flusskontrollierte Beatmung.

Bei der volumenkontrollierten Beatmung wird dem Patienten innerhalb einer vorgegebenen Inspirationszeit ein festgelegtes Atemvolumen appliziert. Das Atemvolumen errechnet sich beispielsweise aus Atemzugvolumen und Atemfrequenz. Bei dieser Beatmungsform versucht das eingesetzte Gerät also im Wesentlichen, das eingestellte Volumen zu applizieren.

Im Gegensatz dazu wird bei der druckunterstützten Beatmung der Atemantrieb des Patienten berücksichtigt. Wenn der Patient einatmet, wird der Atmungsvorgang unterstützt und die Atemarbeit dadurch erleichtert. Bei der druckunterstützten Beatmung werden in der Regel ein unteres Druckniveau und ein oberes Druckniveau bestimmt. Meistens sind diese beiden Drücke variabel einstellbar. Aus der Menge an Luft, die bis zum Erreichen des oberen Drucks abgegeben wurde, ergibt sich dann das Atemzugvolumen.

Allerdings hat sich herausgestellt, dass die Steuerung bzw. Regelung bei Geräten zur druckkontrollierten oder volumenkontrollierten Beatmung sehr aufwendig oder stark verbesserungswürdig ist. Bei der druckkontrollierten oder volumenkontrollierten Beatmung ist, systembedingt, der Anteil an Pendelluft relativ groß; wobei Pendelluft solche Luft ist, die durch die Beatmung (und Atmung) hin- und herbewegt wird und nicht am Gasaustausch teilnimmt.

Die erfindungsgemäße flusskontrollierte Beatmung spült hingegen die oberen Atemwege zumindest teilweise aus und vermindert so den Anteil der Pendelluft.

Demgegenüber ist es die Aufgabe der vorliegenden Erfindung, eine flusskontrollierte Beatmung zu ermöglichen. Vorzugsweise sollen eine verbesserte Steuerbarkeit der Beatmungsgeräte sowie auch eine zuverlässigere Überwachung der Atemtätigkeit des Patienten ermöglicht werden. Insbesondere sollen verbesserte Möglichkeiten zur Verhinderung der Austrocknung der Schleimhäute bereitgestellt werden.

Diese Aufgabe wird gelöst mit einem System des Anspruchs 1 sowie mit einem Verfahren gemäß Anspruch 36. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße System dient zur Atemgasversorgung bzw. Beatmung eines Patienten. Das System umfasst wenigstens eine Beatmungseinrichtung mit wenigstens einer Atemgasquelle zur Erzeugung einer Atemgasströmung. Das System umfasst wenigstens eine Steuereinrichtung, welche dazu geeignet und ausgebildet ist, mittels der Beatmungseinrichtung wenigstens einen definierten Atemgasfluss für eine Beatmung zu erzeugen und vorzugsweise auf eine Sollgröße und insbesondere auf eine bestimmte Flussrate zu regeln. Dabei ist die Steuereinrichtung dazu geeignet und ausgebildet, mittels wenigstens einer Sensoreinrichtung wenigstens einen für einen Druck charakteristischen Parameter zu überwachen. Der Parameter charakterisiert denjenigen Druck, mit welchem die Beatmungseinrichtung die Atemgasströmung zur Aufrechterhaltung des definierten Atemgasflusses beaufschlagt, um eine Atemtätigkeit des Patienten auszugleichen und insbesondere auszuregeln. Dabei ist die Steuereinrichtung insbesondere dazu geeignet und ausgebildet, einen Verlauf des Parameters über die Zeit als einen Druckverlauf zu registrieren.

Das erfindungsgemäße System bietet viele Vorteile. Einen erheblichen Vorteil bietet die Überwachung und Registrierung des Parameters und dass dazu derjenige Druck überwacht wird, den die Beatmungseinrichtung zum Ausgleichen der Atemtätigkeit einstellen muss. Dadurch können die Beatmung und auch die Atemtätigkeit des Patienten besonders unaufwendig und zugleich sehr zuverlässig überwacht werden. Zudem wird dadurch eine besonders zuverlässige und zugleich konstruktiv unaufwendige Möglichkeit zur Regelung der Beatmungseinrichtung in Abhängigkeit der Atemtätigkeit des Patienten ermöglicht.

Die Steuereinrichtung ist vorzugsweise dazu geeignet und ausgebildet, aus dem Druckverlauf (unter Berücksichtigung des Druckverlaufs) wenigstens eine für die Atemtätigkeit des Patienten charakteristische Kenngröße zu bestimmen. Eine solche Kenngröße kann beispielsweise das Atemzugvolumen, die Atemzugdauer, die Atemfrequenz oder dergleichen sein. Die Kenngröße kann auch ein Apnoe-Hypopnoe-Index (AHI) sein. Besonders bevorzugt ist die Kenngröße eine Atemphase, sodass vorzugsweise ein Ausatmen und Einatmen erkannt und voneinander unterschieden wird. Das bietet eine besonders zuverlässige Überwachung der Beatmung. Zudem kann eine solche Kenngröße besonders vorteilhaft zur Regelung der Beatmung eingesetzt werden.

Vorzugsweise ist die Steuereinrichtung dazu geeignet und ausgebildet, eine Einatmung (die Atemphase des Einatmens) dadurch zu erkennen, dass der zur Aufrechterhaltung des definierten Atemgasflusses benötigte Druck über die Zeit abnimmt und insbesondere um ein definiertes Maß abnimmt und/oder sich um ein definiertes Maß ändert. Insbesondere zeigt der Druckverlauf zur Erkennung der Einatmung eine negative Steigung. Es ist möglich, dass dazu eine Auswertung von Ableitungen des Druckverlaufs erfolgt und/oder andere Analysetechniken für Funktionen herangezogen werden. Möglich ist auch, dass der zur Aufrechterhaltung des definierten Atemgasflusses benötigte Druck absinken und/oder unter einen definierten Schwellenwert fallen muss, damit eine Einatmung erkannt wird. Insbesondere sinkt der Druck dazu gegenüber wenigstens einem zuvor registrierten Wert und/oder einem zuvor registrierten Druckverlauf um ein bestimmtes Maß ab. Es ist möglich, dass der Druckverlauf eine definierte Druckdifferenz aufweisen muss, um die Einatmung zu erkennen.

Bevorzugt ist auch, dass die Steuereinrichtung dazu geeignet und ausgebildet ist, eine Ausatmung (die Atemphase des Ausatmens) dadurch zu erkennen, dass der zur Aufrechterhaltung des definierten Atemgasflusses benötigte Druck über die Zeit zunimmt und insbesondere um ein definiertes Maß zunimmt und/oder sich um ein definiertes Maß ändert. Insbesondere zeigt der Druckverlauf zur Erkennung der Ausatmung eine positive Steigung. Es ist möglich, dass dazu eine Auswertung von Ableitungen des Druckverlaufs erfolgt und/oder andere Analysetechniken für Funktionen herangezogen werden. Möglich ist auch, dass der zur Aufrechterhaltung des definierten Atemgasflusses benötigte Druck steigen und/oder über einen definierten Schwellenwert steigen muss, damit eine Ausatmung erkannt wird. Insbesondere steigt der Druck dazu gegenüber wenigstens einem zuvor registrierten Wert und/oder einem zuvor registrierten Druckverlauf um ein bestimmtes Maß an. Es ist möglich, dass der Druckverlauf eine definierte Druckdifferenz aufweisen muss, um die Ausatmung zu erkennen.

Solche Ausgestaltungen bieten eine besonders zuverlässige und zugleich sensorisch unaufwendige Möglichkeit zur Erkennung der Atemphase bzw. einer Einatmung bzw. Ausatmung. Zudem ist die Erkennung der Einatmung bzw. Ausatmung besonders entscheidend für eine gezielte Regelung der Beatmungseinrichtung unter Berücksichtigung der Atemsituation des Patienten.

Insbesondere erfolgt in Abhängigkeit der erkannten Atemphase eine Messung der Atemfrequenz und/oder eine Steuerung der Sauerstoff-Beimischung (insbesondere inspiratorisch mehr als exspiratorisch) und/oder eine Steuerung der Atemgas-Befeuchtung und -erwärmung (insbesondere inspiratorisch mehr als exspiratorisch) und/oder eine Steuerung des Atemgasflusses (insbesondere inspiratorisch mehr als exspiratorisch (Bilevel-Highflow)). Für die Applikation des Bilevel-Highflow erfolgt eine Steuerung derart, dass eine höhere Flussrate während der Inspiration, zur Steigerung des Tidalvolumens, vorgegeben wird z. B. im Bereich von 40-60 l/min. Die Steuerung erfolgt analog mit einer niedrigeren Flussrate während der Exspiration, um eine Ausspülung des Totraums der oberen Atemwege gerade noch zu gewährleisten, bevorzugt im Bereich 10-40 oder 5 - 38 l/min.

Erfindungsgemäß ist die Steuereinrichtung auch dazu Erkennung einer Diskonnektion (Ablösen des Interfaces von der Nase oder des Interfaces vom Schlauch oder des Schlauches vom Gerät) geeignet und ausgebildet. Die Steuereinrichtung erkennt eine Diskonnektion entweder durch das Auftreten einer langen Apnoen, die beispielsweise länger als eine oder bevorzugt länger als zwei Minuten andauert, oder/und durch eine Absenkung der benötigten Drehzahl / Druck, um die eingestellte Flussrate zu erreichen. Bei Erkennung einer Diskonnektion die Steuereinrichtung so geeignet und ausgebildet zum Auslösen eines Diskonnektions-Alarmes und/oder Abschalten der Sauerstoff-Beimischung und/oder zur Reduktion der Flussrate.

Erfindungsgemäß ist auch vorgesehen, den Highflow-Modus nicht über eine Nasenbrille, sondern über eine normale Atemmaske zu applizieren. In diesem Anwendungsfall wirkt die Lüftereinrichtung der Atemtätigkeit des Patienten entgegen - sie versucht ja, einen gleichbleibenden Fluss zu erzeugen. Dies kann als Trainings-Programm für die Atemmuskeln verwendet werden. Je höher der eingestellte Fluss, desto mehr Widerstand (Training) erfährt der Patient bei der Ausatmung. Je niedriger der eingestellte Fluss, desto mehr Widerstand (Training) erfährt der Patient bei der Einatmung. Die Gesamt-Schwere des Trainings lässt sich erfindungsgemäß von der Steuerung (über die Reglerkoeffizienten der Highflow-Regler) einstellen. Je träger / vorsichtiger die Regelung, desto geringer der Widerstand bei der Atmung (In/Ex gleichermaßen). Je dynamischer / aggressiver die Regelung, desto höher ist der Widerstand. Über Telemonitoring lässt sich die tägliche Dauer des Atemmuskeltrainings erfassen.

In einer besonders vorteilhaften Weiterbildung ist die Steuereinrichtung dazu geeignet und ausgebildet, anhand des Druckverlaufs wenigstens ein Atemereignis zu bestimmen. Die Bestimmung des Atemereignisses erfolgt insbesondere durch Vergleich mit hinterlegten Druckverläufen. Solche Atemereignisse sind beispielsweise Schnarchen, Atemabflachungen und/oder obstruktive Druckspitzen, Apnoen, Hypopnoen oder andere entsprechende Ereignisse.

Es ist möglich und bevorzugt, dass die Steuereinrichtung dazu geeignet und ausgebildet ist, anhand des Druckverlaufs wenigstens ein Atemmuster zu bestimmen. Vorzugsweise ist die Steuereinrichtung dazu geeignet und ausgebildet, das Atemmuster auf wenigstens ein Atemereignis zu untersuchen. Die Untersuchung des Atemmusters erfolgt insbesondere durch Vergleich mit wenigstens einem hinterlegten Atemmuster und/oder mittels wenigstens eines (lernfähigen) Algorithmus. Das Atemmuster entspricht insbesondere einem charakteristischen zeitlichen Verlauf der Atmung und vorzugsweise der darin auftretenden Atemereignisse. Beispielsweise kann durch die Untersuchung eines Atemmusters eine Wacherkennung bzw. Schlaferkennung erfolgen. Ein Atemmuster ist beispielsweise durch die Abfolge von Apnoen und/oder Hypopnoen gekennzeichnet.

Es ist möglich, dass die Steuereinrichtung dazu geeignet und ausgebildet ist, den berechneten Druckverlauf und/oder das berechnete Atemmuster und/oder ein erkanntes Atemereignis auf Plausibilität zu überprüfen. Dazu können bekannte Methoden zur Auswertung von Druckverläufen bzw. Ereignissen im Rahmen von Beatmungen herangezogen werden. Beispielsweise muss ein vorgegebenes Signifikanzniveau erreicht werden, damit ein bestimmtes Atemereignis und/oder Atemmuster erkannt wird.

Insbesondere ist die Steuereinrichtung dazu geeignet und ausgebildet, anhand des Druckverlaufs eine Ansteuerung der Beatmungseinrichtung vorzunehmen, wie sie beispielsweise aus der druckunterstützten bzw. druckkontrollierten Beatmung bekannt ist. In Abhängigkeit des Druckverlaufs ist insbesondere wenigstens einer der nachfolgenden Einstellungen bzw. Regelungen durchführbar: Autostart, Autostopp, Rampe, Wacherkennung, Ereigniserkennung, Statistik, Frequenz, Atemvolumen, Apnoen, Hypopnoen, Ereignissteuerung, Sättigungssteuerung, automatische Steuerung der Flowrate, Teleüberwachung, Nahüberwachung, Steuerung bzw. Einstellung von PSG und/oder SpO2 und/oder TcCO2, Soft-PAP, Sauerstoffbeimischung. Insbesondere ist in Abhängigkeit des Druckverlaufs wenigstens eine aus einer CPAP-, IPAP- oder BiPAP-Beatmung bekannten Steuerung möglich.

Die Steuereinrichtung kann dazu geeignet und ausgebildet sein, abhängig vom Druckverlauf wenigstens eine Benutzerinteraktion und beispielsweise einen Alarm auszugeben. Möglich ist auch, dass die Steuereinrichtung dazu geeignet und ausgebildet ist, den Druckverlauf und insbesondere ein darüber erkanntes Atemereignis und/oder Atemmuster an wenigstens eine Netzwerkeinrichtung zu übertragen. Insbesondere ist das System zum Telemonitoring ausgebildet. Insbesondere ist durch die Steuereinrichtung abhängig vom Druckverlauf eine Therapiestatistik erstellbar und/oder übertragbar.

In allen Ausgestaltungen ist es besonders bevorzugt, dass die Steuereinrichtung dazu geeignet und ausgebildet ist, in Abhängigkeit des Druckverlaufs wenigstens einen Geräteparameter der Beatmungseinrichtung einzustellen und/oder mittels der Beatmungseinrichtung wenigstens einen Beatmungsparameter einzustellen. Insbesondere ist in Abhängigkeit des Druckverlaufs die Beatmungseinrichtung ansteuerbar. Insbesondere ist die Steuereinrichtung dazu geeignet und ausgebildet, den Geräteparameter und/oder Beatmungsparameter als eine Reaktion auf ein Atemereignis und/oder ein Atemmuster einzustellen. Der Geräteparameter umfasst beispielsweise eine Lüfterdrehzahl und/oder eine Befeuchtungsleistung und/oder eine Heizleistung oder dergleichen. Der Geräteparameter kann auch ein Anschalten und/oder Ausschalten und/oder Pausieren der Beatmungseinrichtung umfassen. Der Geräteparameter kann auch Softwareeinstellungen betreffen. Der Beatmungsparameter ist vorzugsweise ein Flow bzw. eine Flussrate und/oder ein Druck und/oder ein anderer für die Beatmung charakteristischer Parameter. Der Geräteparameter und der Beatmungsparameter können gekoppelt sein und/oder sich gegenseitig beeinflussen. Es ist möglich, dass die Geräteparameter zur Umsetzung eines bestimmten Beatmungsparameters eingestellt werden.

Vorzugsweise erfolgt die Einstellung des Geräteparameters und/oder Beatmungsparameters in Abhängigkeit wenigstens eines erkannten Atemmusters und/oder wenigstens eines Atemereignisses. Insbesondere erfolgt die Einstellung der Flussrate des Atemgasflusses in Abhängigkeit des erkannten Atemmusters und/oder Atemereignisses.

In einer vorteilhaften Ausgestaltung ist die Steuereinrichtung dazu geeignet und ausgebildet, mittels der Beatmungseinrichtung wenigstens eine Flussrate des Atemgasflusses in Abhängigkeit einer Sättigung des Atemgasflusses mit Sauerstoff einzustellen. Möglich ist auch, dass wenigstens ein anderer Beatmungsparameter in Abhängigkeit der Sauerstoffsättigung einstellbar ist. Insbesondere wird die Sauerstoffsättigung sensorisch überwacht. Insbesondere ist die Sauerstoffsättigung sensorisch erfassbar. Möglich ist auch, dass die Flussrate des Atemgasflusses in Abhängigkeit einer vom System vorgenommenen und/oder fest eingestellten Sauerstoffbeimischung einstellbar ist.

Die Steuereinrichtung ist vorzugsweise dazu geeignet und ausgebildet, in Abhängigkeit des Druckverlaufs eine Sauerstoffbeimischung in die Atemgasströmung einzustellen. Vorzugsweise ist die Steuereinrichtung dazu geeignet und ausgebildet, die Sauerstoffbeimischung atemphasenabhängig zu modulieren und insbesondere in Abhängigkeit des Druckverlaufs atemphasenabhängig zu modulieren. Insbesondere ist die Sauerstoffbeimischung in Abhängigkeit des Druckverlaufs gezielt vergrößerbar und/oder verringerbar. Insbesondere ist zur Sauerstoffbeimischung wenigstens eine gezielt öffenbare bzw. verschließbare Sauerstoffquelle vorgesehen. Die Sauerstoffbeimischung kann bis zu 100 % betragen. Möglich ist auch, dass die Sauerstoffbeimischung deaktiviert ist bzw. 0 % beträgt. Die Sauerstoffbeimischung kann beispielsweise bis zu 300 l/min betragen.

Insbesondere erfolgt die Sauerstoffbeimischung nach wenigstens einer der folgenden Vorgaben: Sauerstoffanteil halten, Sauerstoff Absolutfluss halten, bedarfsgesteuert aus gemessenen oder geschätzten Patientenbedarf. Insbesondere ist für die Sauerstoffbeimischung wenigstens eine sensorbasierte Regelung vorgesehen. Die sensorbasierte Regelung umfasst insbesondere eine Regelung nach SpO2 und/oder TcCO2 und/oder andere Regelvorgaben.

Besonders bevorzugt wird die Sauerstoffbeimischung und/oder ein Sauerstoffgehalt im Atemgasfluss um ein bestimmtes Maß verringert, wenn eine Flussrate des Atemgasflusses erhöht wird. Eine solche inverse Anpassung hat sich gemäß klinischen Erfahrungen als besonders vorteilhaft erwiesen. Durch die bei der flusskontrollierten Beatmung entsprechend große Flussrate wird eine bessere Sauerstoffaufnahme erreicht, sodass die Beimischung entsprechend verringert werden kann. Insbesondere geht eine Steigerung der Flussrate des Atemgasflusses mit einer Abnahme der Sauerstoffbeimischung bzw. des Sauerstoffgehalts im Atemgasfluss einher. Insbesondere ist wenigstens eine Zuordnungsfunktion hinterlegt, welche eine umgekehrte Proportionalität zwischen der Sauerstoffbeimischung und der Flussrate beschreibt.

Insbesondere erfolgt die Sauerstoffbeimischung nur während einer Einatmung. Insbesondere erfolgt die Sauerstoffbeimischung nicht während einer Ausatmung. Möglich und bevorzugt ist, dass die Sauerstoffbeimischung während einer Einatmung gegenüber einer Ausatmung erhöht wird. Dabei werden die Einatmung und Ausatmung vorzugsweise wie zuvor beschrieben anhand des Druckverlaufs erkannt. Eine solche verstärkte Beimischung während der Inspiration bietet viele Vorteile. Möglich ist aber auch eine sensorbasierte Regelung.

Die Anmelderin behält sich vor, ein System zur Atemgasversorgung bzw. Beatmung eines Patienten zu beanspruchen. Ein solches System umfasst wenigstens eine Beatmungseinrichtung mit wenigstens einer Atemgasquelle zur Erzeugung einer Atemgasströmung. Das System umfasst wenigstens eine Steuereinrichtung, welche dazu geeignet und ausgebildet ist, mittels der Beatmungseinrichtung wenigstens einen definierten Atemgasfluss für eine volumenkontrollierte Beatmung zu erzeugen und vorzugsweise auf eine Sollgröße und insbesondere auf eine bestimmte Flussrate zu regeln. Dabei umfasst das System wenigstens eine Befeuchtungseinrichtung zur insbesondere gezielten Befeuchtung der Atemgasströmung und/oder wenigstens eine Heizeinrichtung zur insbesondere gezielten Beheizung der Atemgasströmung. Ein solches System ist vorzugsweise wie das zuvor beschriebene System ausgebildet. Vorzugsweise ist auch das zuvor beschriebene System derart ausgebildet.

Die Befeuchtungseinrichtung umfasst beispielsweise einen Vernebler und/oder Anfeuchter und/oder Befeuchter und/oder Verdampfer. Es ist möglich, dass die Heizeinrichtung auch zum Abkühlen der Atemgasströmung geeignet ist. Es ist möglich, dass die Befeuchtungseinrichtung auch zur Entfeuchtung der Atemgasströmung geeignet ist.

Insbesondere ist die Steuereinrichtung dazu geeignet und ausgebildet, in Abhängigkeit des Druckverlaufs eine Befeuchtung und/oder Beheizung der Atemgasströmung einzustellen und insbesondere auf eine Zieltemperatur und/oder Zielfeuchte des Atemgasflusses zu regeln. Die Zieltemperatur und/oder Zielfeuchte wird insbesondere am Patienten und/oder in der Nähe des Patienten gemessen. Beispielsweise wird die Zieltemperatur und/oder Zielfeuchte an einem Ausgangsbereich einer Schlauchverbindung zum Patienten und/oder im Bereich einer Atemschnittstelle gemessen. Möglich ist auch eine Messung an einer anderen Stelle, beispielsweise an einem Geräteausgang bzw. an einem Ausgang der Beatmungseinrichtung. Insbesondere ist zum Erfassen der Zieltemperatur und/oder Zielfeuchte wenigstens eine Sensoreinrichtung mit entsprechenden Sensoren vorgesehen. Die Befeuchtung erfolgt insbesondere mit der zuvor beschriebenen Befeuchtungseinrichtung. Die Beheizung erfolgt insbesondere mit der zuvor beschriebenen Heizeinrichtung.

Vorzugsweise ist mittels der Steuereinrichtung eine atemphasengesteuerte Befeuchtung und/oder Beheizung einstellbar. Dabei wird die Atemphase vorzugsweise wie zuvor beschrieben anhand des Druckverlaufs ermittelt.

Die Steuereinrichtung ist insbesondere dazu geeignet und ausgebildet, die Befeuchtung und/oder Beheizung in Abhängigkeit einer Flussrate des Atemgasflusses einzustellen. Eine solche Anpassung der Befeuchtung bzw. Beheizung an die Flussrate ist besonders vorteilhaft, da bei den entsprechend hohen Flussraten des Highflow trockene bzw. kalte Strömungen oft als besonders unangenehm empfunden werden.

Die Steuereinrichtung ist vorzugsweise dazu geeignet und ausgebildet, die Befeuchtung und/oder Beheizung in Abhängigkeit wenigstens einer sensorisch erfassten Größe einzustellen. Dabei ist die Größe vorzugsweise aus einer Gruppe von Größen entnommen, wobei die Gruppe wenigstens umfasst: Umgebungstemperatur, Umgebungsfeuchte, Temperatur im System, Feuchte im System, Schlauchtemperatur, Temperatur an einer Patientenschnittstelle, Feuchte am Schlauchende und/oder an einer Patientenschnittstelle. Die Temperatur bzw. Feuchte im System kann beispielsweise im Bereich der Heizeinrichtungen insbesondere an einem Heizstab und/oder Platte und/oder Sensor abgegriffen werden. Die Temperatur bzw. Feuchte im System kann auch innerhalb der Befeuchtungseinrichtung und/oder in/an einem Gehäuse und/oder an einer Lüftereinrichtung und insbesondere einem Lüfterausgang abgegriffen werden.

In einer besonders vorteilhaften Ausgestaltung ist die Steuereinrichtung dazu geeignet und ausgebildet, durch die Befeuchtung und/oder Beheizung verursachte Flowfehler und/oder andere Abweichung mittels der Beatmungseinrichtung wenigstens näherungsweise zu kompensieren. Insbesondere sind Flowfehler zwischen 0,1 % und 10 % kompensierbar. Insbesondere sind Flowfehler von einigen Prozent kompensierbar. Insbesondere sind durch die Befeuchtung bzw. Verdampfung von Wasser verursachte Flowfehler kompensierbar.

In einer besonders vorteilhaften Weiterbildung ist die Steuereinrichtung dazu geeignet und ausgebildet, die für die Beatmung vorgesehene Flussrate des Atemgasflusses so lange zu reduzieren, bis ein Maß für die Feuchte des Atemgasflusses einen Schwellenwert erreicht und/oder bis die Temperatur des Atemgasflusses einen Schwellenwert erreicht. Dabei ist die für die Beatmung vorgesehene Flussrate des Atemgasflusses insbesondere um ein hinterlegtes Maß und/oder auf einen hinterlegten Wert reduzierbar. Insbesondere wird dann bei Erreichen und/oder Überschreiten des Schwellenwertes die für die Beatmung vorgesehene Flussrate vollständig bereitgestellt.

Besonders bevorzugt wird die für die Beatmung vorgesehene Flussrate des Atemgasflusses erst dann bereitgestellt, wenn ein Maß für die Feuchte des Atemgasflusses und/oder die Temperatur des Atemgasflusses einen Schwellenwert erreicht. Vorzugsweise ist für die Flussrate des Atemgasflusses eine feuchtegesteuerte Rampe hinterlegt.

In einer besonders vorteilhaften Weiterbildung umfasst das System eine Befeuchtungseinrichtung zur Befeuchtung der Atemgasströmung und wenigstens eine Heizeinrichtung zur Beheizung der Atemgasströmung wobei die Heizeinrichtung dazu geeignet und ausgebildet ist die Atemgasströmung (zumindest in der Inspiration) auf 36 - 38 °C zu erwärmen und wobei die Befeuchtungseinrichtung dazu geeignet und ausgebildet ist die Atemgasströmung mit einer relativen Feuchtigkeit im Bereich von 90 - 100%, bevorzugt 95 - 99%, zu befeuchten.

Solche Ausgestaltungen bieten eine besonders komfortable Beatmung, da die Befeuchtung und Beheizung einen erheblichen Einfluss auf den Komfort der Beatmung haben. Besonders vorteilhaft ist die Einstellung der für die Beatmung vorgesehene Flussrate erst dann, wenn eine Befeuchtung sicher gegeben ist, da eine Highflow-Beatmung ohne ausreichende Befeuchtung unter Umständen schmerzhaft sein kann. Es ist von Vorteil, zunächst bei einer geringeren Flussrate und einer möglichst hohen Feuchte bzw. 100 % Feuchte aufzuheizen und anschließend erst die Flussrate zu erhöhen.

In einer vorteilhaften Ausgestaltung ist im Nachlauf einer Beatmung wenigstens ein Trocknungsmodus durchführbar. Insbesondere erfolgt dabei ein Trocknen einer Schlaucheinrichtung und insbesondere eines Beatmungsschlauches. Insbesondere wird dabei Kondenswasser gezielt entfernt. Der Trocknungsmodus kann mittels der Heizeinrichtung und/oder Befeuchtungseinrichtung durchführbar sein.

Es ist möglich, dass wenigstens ein Kondensationsschutz vorgesehen ist. Dazu wird beispielsweise 95 % relative Feuchte bei 37 °C gehalten. Möglich sind auch andere Kombinationen von Feuchte und Temperatur.

Die Befeuchtungseinrichtung ist insbesondere dazu geeignet und ausgebildet, dass für die Befeuchtung vorgesehene Wasser vor einem Aufheizen aus wenigstens einem Reservoir zu entnehmen und separat zum Reservoir zu erhitzen. Dabei ist Wasser insbesondere synonym zu anderen zur Befeuchtung geeigneten Flüssigkeiten zu verstehen. Das Vereinzeln des Wassers kann beispielsweise im Prinzip einer Vogeltränke erfolgen. Zur Vereinzelung werden insbesondere Kapillareffekte genutzt. Es ist möglich, dass die Heizeinrichtung wenigstens eine Heizplatte umfasst, welche wenigstens ein Heizelement und beispielsweise eine Heizplatte umfasst, welches in einem Schwimmer mit kleinem Becken angeordnet ist. Das für die Befeuchtung vorgesehene Wasser kann auch vernebelt werden. Dazu ist beispielsweise ein Meshvernebler und/oder Düsenvernebler und/oder Rotationsvernebler und/oder ein Vernebler mit Heizschlauch vorgesehen. Dabei ist vorzugsweise eine entsprechende Regelung bzw. Sicherung ausgebildet, welche sicherstellt, dass kein Nebel in den Patienten gelangt.

Die Anmelderin behält sich vor, ein System zur Atemgasversorgung bzw. Beatmung eines Patienten zu beanspruchen. Ein solches System umfasst wenigstens eine Beatmungseinrichtung mit wenigstens einer Atemgasquelle zur Erzeugung einer Atemgasströmung. Das System umfasst wenigstens eine Steuereinrichtung, welche dazu geeignet und ausgebildet ist, mittels der Beatmungseinrichtung wenigstens einen definierten Atemgasfluss für eine volumenkontrollierte Beatmung zu erzeugen und vorzugsweise auf eine Sollgröße und insbesondere auf eine bestimmte Flussrate zu regeln. Dabei umfasst das System wenigstens eine Schlaucheinrichtung mit wenigstens einem an die Beatmungseinrichtung koppelbaren Beatmungsschlauch, mit dem die Atemgasströmung einer Patientenschnittstelle zuführbar ist. Ein solches System ist vorzugsweise wie eines der zuvor beschriebenen Systeme ausgebildet. Vorzugsweise sind auch die zuvor beschriebenen Systeme derart ausgebildet.

Die Schlaucheinrichtung kann wenigstens einen beheizbaren Beatmungsschlauch umfassen. Insbesondere der beheizbare Beatmungsschlauch wenigstens einen Teil einer Sensoreinheit zur Erfassung wenigstens einer für die Feuchte und/oder Temperatur charakteristischen Größe. Insbesondere ist der Teil der Sensoreinheit innerhalb des Beatmungsschlauches untergebracht und/oder in diesen integriert. Dieser Teil der Sensoreinheit kann auch wenigstens teilweise oder auch vollständig außerhalb des Beatmungsschlauches angeordnet sein. Dann ist dieser Teil der Sensoreinheit vorzugsweise außen an dem Beatmungsschlauch angeordnet. Die mittels der Sensoreinheit erfassbare Größe ist insbesondere für die Steuerung bzw. Regelung der Beheizung des Schlauches einsetzbar. Insbesondere erfolgt über die derart erfasste Größe Regelung der Beheizung des Schlauches. Es ist möglich, dass zur Beheizung des Schlauches wenigstens eine weitere Größe herangezogen wird.

Vorzugsweise umfasst die Schlaucheinrichtung wenigstens einen graduell über seine Länge beheizbaren Beatmungsschlauch. Insbesondere ist ein Beheizungsgradient bzw. Temperaturgradient über die Schlauchlänge einstellbar. Insbesondere weist der Beatmungsschlauch wenigstens einen stärker beheizbaren ersten Abschnitt auf, welcher zur Verdampfung von Wasser geeignet und ausgebildet ist.

Insbesondere umfasst der Beatmungsschlauch wenigstens einen zweiten Abschnitt, welcher gegenüber dem ersten Abschnitt geringer beheizt wird, sodass in dem zweiten Abschnitt eine gezielte Kompensation der Wärmeverluste in Bezug auf das verdampfte Wasser erreicht wird.

In einer besonders vorteilhaften Ausgestaltung umfasst die Schlaucheinrichtung wenigstens einen beheizbaren Beatmungsschlauch mit wenigstens einer von dem Beatmungsschlauch trennbaren Heizung. Dabei ist die Heizung bei einem Schlauchwechsel mit einem neuen Beatmungsschlauch bestimmungsgemäß ausrüstbar und weiterverwendbar. Das bietet eine besonders wirtschaftliche und nachhaltige Verwendung eines beheizbaren Beatmungsschlauches. Möglich ist auch, dass die Sensoreinheit des Beatmungsschlauches von diesem trennbar und nach einem Schlauchwechsel bestimmungsgemäß weiter verwendbar ist.

Die Schlaucheinrichtung weist vorzugsweise wenigstens einen Beatmungsschlauch aus einem Wasser ausleitenden Material auf. Beispielsweise ist das Material analog zu einer Klimafolie im Hausbau und/oder zu einem Klimatextil der Funktionsbekleidung ausgebildet.

Die Schlaucheinrichtung kann wenigstens einen wärmeisolierten Beatmungsschlauch umfassen. Insbesondere ist der Beatmungsschlauch doppelwandig ausgebildet. Insbesondere sind zwischen wenigstens zwei Wandungen Lufteinschlüsse und/oder vakuumierte Bereiche vorgesehen. Möglich ist auch, dass der Beatmungsschlauch von einem wärmeisolierenden Werkstoff umgeben ist. Insbesondere ist eine Wärmeisolierung gegenüber der Umgebung vorgesehen.

In allen Ausgestaltungen des Systems ist es besonders bevorzugt, dass die Steuereinrichtung dazu geeignet und ausgebildet ist, mittels der Beatmungseinrichtung durch den definierten Atemgasfluss innerhalb einer vorgegebenen Inspirationszeit wenigstens ein festgelegtes Atemvolumen zu applizieren. Insbesondere errechnet sich das Atemminutenvolumen dabei aus dem Atemzugvolumen und der Atemfrequenz.

Die Steuereinrichtung ist vorzugsweise dazu geeignet und ausgebildet, für den definierten Atemgasfluss wenigstens einen hinterlegten Maximaldruck zu berücksichtigen. Der Maximaldruck kann fest vorgegeben sein und/oder dynamisch anpassbar sein. Insbesondere erfolgt eine dynamische Anpassung des Maximaldrucks in Abhängigkeit des Druckverlaufs. Möglich ist auch, dass der Maximaldruck manuell einstellbar ist. Es ist möglich, dass ein Überschreiten des Maximaldrucks nur unter besonderen Sicherheitsvorkehrungen möglich ist, beispielsweise durch die Eingabe eines Passwortes bzw. einer Benutzerkennung oder dergleichen. Da die Beatmungseinrichtung bei der flusskontrollierten Beatmung in erster Linie versucht, das eingestellte Volumen zu applizieren, können bei dieser Beatmungsform mitunter hohe Spitzendrücke entstehen. Daher ist eine solche Ausgestaltung eine besonders sichere Möglichkeit, unvorteilhafte Spitzendrücke zuverlässig zu verhindern. Insbesondere ist wenigstens ein Maximaldruck als Alarmgrenze hinterlegt.

Insbesondere weist der definierte Atemgasfluss eine Flussrate im Bereich von 0 bis 90 l/min und bevorzugt 1 bis 80 l/min und besonders bevorzugt 2 bis 60 l/min auf. Möglich sind auch andere Flussraten. Insbesondere ist die Beatmungseinrichtung auf eine solche Flussrate einstellbar. Der definierte Atemgasfluss kann, per auswählbarer Voreinstellung, eine Flussrate für Säuglinge im Bereich von 1 - 10 l/min aufweisen. Insbesondere ist mit einer solchen Flussrate bestimmungsgemäß eine Beatmung durchführbar. Insbesondere ist ein Atemgasfluss mit einer Flussrate von 0 bis 300 l/min einstellbar.

In allen Ausgestaltungen ist das System dazu geeignet und ausgebildet, bestimmungsgemäß mit einer Nasenbrille als Patientenschnittstelle betrieben zu werden. Insbesondere ist eine offene Nasenbrille vorgesehen. Insbesondere ist die Atemgasströmung mit einer Nasenbrille dem Patienten applizierbar. Insbesondere ist an die Beatmungseinrichtung wenigstens eine Nasenbrille anschließbar. Insbesondere ist die Nasenbrille mit der Schlaucheinrichtung verbindbar. Möglich ist auch, dass die Schlaucheinrichtung fest und/oder lösbar mit einer Nasenbrille verbunden ist. Insbesondere ist der Beatmungsschlauch an eine Nasenbrille koppelbar. Insbesondere umfasst das System wenigstens eine Nasenbrille. Eine solche Nasenbrille eignet sich zur Highflow-Beatmung besonders vorteilhaft und bietet dem Patienten einen besonders hohen Komfort. Möglich ist auch, dass das System mit einer anderen geeigneten Patientenschnittstelle und zum Beispiel mit einem Tracheostomieanschluss betreibbar ist.

In einer vorteilhaften Weiterbildung weist das System eine Nasenbrille mit einem Stutzen für jedes Nasenloch auf, wobei jeder Stutzen zumindest teilweise in das jeweilige Nasenloch einführbar ist und wobei jeder Stutzen einen Durchmesser aufweist, der so bemessen ist, dass das jeweilige Nasenloch bei eingeführtem Stutzen nicht dicht verschlossen ist.

Jeder Stutzen kann beispielsweise einen Durchmesser aufweisen, der weniger als 9/10, bevorzugt weniger als 8/10 und mehr als 6/10, des Durchmessers des jeweiligen Nasenloches beträgt.

Das erfindungsgemäße Verfahren dient zum Betreiben eines Systems, wie es zuvor beschrieben wurde. Insbesondere sind die zuvor beschriebenen Systeme dazu geeignet und ausgebildet, nach dem erfindungsgemäßen Verfahren betrieben zu werden.

Der Parameter ist insbesondere ein Druck. Der Parameter, welcher von der Sensoreinrichtung überwacht wird, ist insbesondere der Druck, mit dem die Beatmungseinrichtung die Atemgasströmung beaufschlagen muss, um den definierten Atemgasfluss gegenüber der Atemtätigkeit des Patienten aufrechtzuerhalten und insbesondere auf einer bestimmten Flussrate bzw. Soll-Flussrate und/oder auf wenigstens einer anderen für den Atemgasfluss charakteristischen Kenngröße zu halten. Der Parameter beschreibt insbesondere den Druck der Atemgasströmung in der Beatmungseinrichtung und/oder an einem Ausgang der Beatmungseinrichtung und insbesondere an einer Schnittstelle zur Verbindung mit einer Schlaucheinrichtung und/oder einer Patientenschnittstelle. Der Parameter beschreibt insbesondere eine Druckanpassung, welche notwendig ist, um den Istwert des Atemgasflusses auf einen geforderten Sollwert zu bringen. Möglich ist auch, dass der Parameter wenigstens eine andere für einen Druck charakteristische Größe darstellt. Dann ist der Parameter beispielsweise eine Lüfterdrehzahl oder dergleichen.

Die Sensoreinrichtung umfasst insbesondere wenigstens einen Sensor zur Erfassung des Parameters. Die Sensoreinrichtung umfasst insbesondere wenigstens einen Drucksensor und/oder wenigstens einen Drehzahlsensor und/oder wenigstens einen Flowsensor und/oder einen anderen geeigneten Sensor.

Insbesondere ist die Steuereinrichtung dazu geeignet und ausgebildet, den Druck zu ermitteln, welchen die Beatmungseinrichtung aufbringen muss, um den definierten Atemgasfluss konstant zu halten. Insbesondere ist die Steuereinrichtung dazu geeignet und ausgebildet, den Druck zu ermitteln, welchen die Beatmungseinrichtung aufbringen muss, um den durch eine Atemtätigkeit beeinflussten Atemgasfluss auf einen definierten Sollwert zu halten. Insbesondere ist die Steuereinrichtung dazu geeignet und ausgebildet, den definierten Atemgasfluss dadurch auf eine bestimmte Flussrate zu regeln, dass mittels der Beatmungseinrichtung eine Druckanpassung und/oder wenigstens eine Drehzahlanpassung einer Lüftereinrichtung vorgenommen wird. Der Druck der Atemgasströmung wird insbesondere durch eine Drehzahl einer Lüftereinrichtung eingestellt. Möglich ist auch, dass der Druck durch ein gezieltes Öffnen bzw. Schließen einer Druckgasquelle eingestellt wird. Die Steuereinrichtung ist insbesondere dazu geeignet und ausgebildet, den definierten Atemgasfluss mittels Druckanpassungen zu regeln. Die Steuereinrichtung kann insbesondere Druckanpassungen vornehmen, um den definierten Atemgasfluss auf einer bestimmten Flussrate zu halten und dabei vorzugsweise die Atemtätigkeit des Patienten auszuregeln.

Mit dem erfindungsgemäßen System ist insbesondere eine flusskontrollierte- oder Highflow-Beatmung durchführbar. Insbesondere ist die Beatmungseinrichtung mittels der Steuereinrichtung ansteuerbar. Insbesondere ist die Beatmungseinrichtung mittels der Steuereinrichtung zur Vorgabe eines definierten Atemgasflusses ansteuerbar.

Der definierte Atemgasfluss ist insbesondere ein Permanentfluss. Der definierte Atemgasfluss ist insbesondere durch eine vorgegebene Flussrate definiert. Der definierte Atemgasfluss kann auch durch wenigstens eine andere für einen Volumenstrom charakteristische Kenngröße definiert sein. Im Rahmen der vorliegenden Erfindung wird die Flussrate auch als Flowrate bzw. Flow bezeichnet. Der Atemgasfluss kann auch als Flow bzw. Atemgasflow bezeichnet werden. Der definierte Atemgasfluss beschreibt insbesondere die Menge des dem Patienten bereitgestellten bzw. des in den Patienten einfließenden Atemgases bezogen auf die Zeit. Die flusskontrollierte- und Highflow-Beatmung werden vorliegend als Synonyme verwendet.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine rein schematische Darstellung eines erfindungsgemäßen Systems in einer perspektivischen Ansicht;
- Fig. 2: ein stark schematisiertes Schaubild zur Funktionsweise des Systems; und
- Fug. 3: ein weiteres stark schematisiertes Schaubild zur Funktionsweise des Systems.

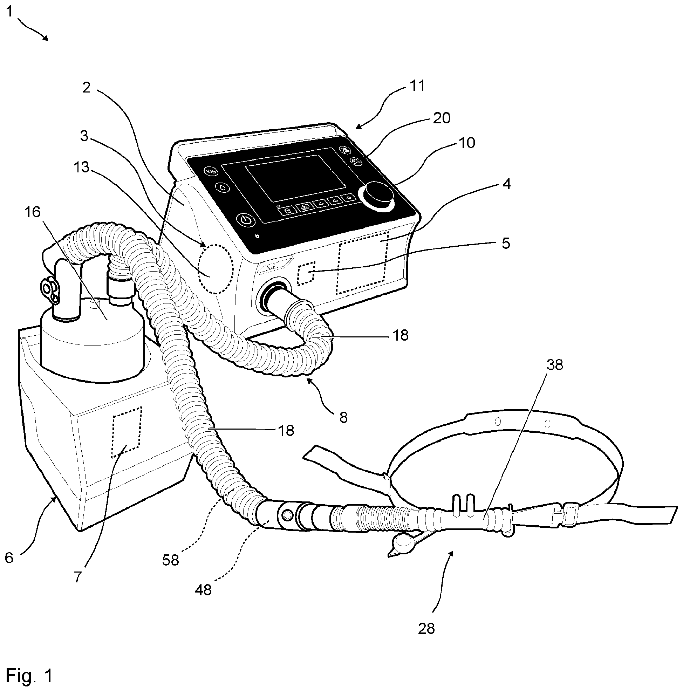

Die Figur 1 zeigt ein erfindungsgemäßes System 1 zur Atemgasversorgung bzw. Beatmung mit wenigstens einer Beatmungseinrichtung 2, welche hier durch ein Beatmungsgerät 11 bereitgestellt wird. Das hier gezeigte System 1 wird nach dem erfindungsgemäßen Verfahren betrieben. Die Beatmungseinrichtung 2 ist mit einer Atemgasquelle 3 zur Erzeugung einer Atemgasströmung ausgestattet, welche hier durch eine Lüftereinrichtung 13 im Inneren des Gehäuses bereitgestellt wird.

Die Lüftereinrichtung 13 erzeugt mittels eines Lüfters die Atemgasströmung, welche über eine an die Beatmungseinrichtung 2 gekoppelte Schlaucheinrichtung 8 dem Patienten zugeführt wird. Beispielsweise ist ein Elektromotor mit Lüfterrad vorgesehen. Zusätzlich oder alternativ kann als Atemgasquelle 3 auch eine Druckgasquelle vorgesehen sein.

Das Beatmungsgerät 11 umfasst hier eine Bedieneinrichtung 10 und eine Anzeigeeinrichtung 20. Dabei können auch Kombinationen von Bedieneinrichtung 10 und Anzeigeeinrichtung 20 vorgesehen sein, beispielsweise in Form einer berührungsempfindlichen Anzeigefläche bzw. Touch-Screens.

Das System 1 ist für eine flusskontrollierte Beatmung bzw. Highflow-Beatmung ausgebildet. Die Beatmungseinrichtung 2 wird von einer hier nicht sichtbar innerhalb des Gehäuses angeordneten Steuereinrichtung bzw. Steuereinheit 4 angesteuert. Für die flusskontrollierte Beatmung stellt die Steuereinrichtung 4 die Beatmungseinrichtung 2 auf einen definierten Atemgasfluss ein. Dadurch wird dem Patienten innerhalb einer vorgegebenen Inspirationszeit ein festgelegtes Atemvolumen appliziert. In einer Ausgestaltung kann das System 1 auch zur druckunterstützten bzw. druckkontrollierten Beatmung, bei definiertem Fluss, einsetzbar sein.

Das System 1 ist hier mit einer für die flusskontrollierte Beatmung angepassten bzw. optimierten Schlaucheinrichtung 8 ausgestattet. Beispielsweise wird ein Atemgasfluss mit einer Flussrate von 0 bis 300 l/min eingestellt. Dazu ist ein Beatmungsschlauch 18 vorgesehen, welcher mit der Atemgasquelle 3 und einer Patientenschnittstelle bzw. Patienteninterface 28 gekoppelt ist.

Die Patientenschnittstelle 28 ist hier als eine offene Nasenbrille 38 ausgebildet. Die Nasenbrille 38 kann auch als Nasenkanüle bezeichnet werden. Vorteilhaft kann die Schlaucheinrichtung auch mit einem Tracheostomieanschluss verwendet werden. Eine solche Nasenbrille 38 ist in der Regel erheblich komfortabler für den Patienten als beispielsweise eine feste Atemmaske und ein besonderer Vorteil der flusskontrollierten Beatmung. Dabei wird die Nasenbrille 38 lose in die Nase gehängt und mit einem entsprechend hohen vordefinierten Atemgasfluss (Highflow oder auch Permanentfluss) durchspült. Dadurch wird das Kohlendioxid aus den oberen Atemwegen vor der Einatmung ausgewaschen und es entsteht ein kleiner positiver Überdruck, sodass vor allem die Einatmung besonders angenehm unterstützt werden kann. Die Nasenbrille erlaubt eine ständige Leckage während die Atemmaske eine Leckage im Wesentlichen vermeidet.

Damit die Schleimhäute durch den permanenten Luftstrom nicht ausgetrocknet werden, wird die Atemgasströmung hier gezielt befeuchtet und beheizt. Dazu ist das System 1 hier mit einer Befeuchtungseinrichtung 6 und einer Heizeinrichtung 7 ausgestattet. Die Heizeinrichtung 7 ist in der Befeuchtungseinrichtung 6 integriert. Die Steuereinrichtung 4 aktiviert zusätzlich zumindest zeitweise die Befeuchtungseinrichtung 6 und oder die Heizeinrichtung 7 zur Anfeuchtung bzw. Erwärmung des Atemgases.

Der Beatmungsschlauch 18 ist zweiteilig ausgebildet und erstreckt sich mit einem ersten Teil von der Beatmungseinrichtung 2 zu der Befeuchtungseinrichtung 6. Ein zweiter Teil des Beatmungsschlauchs 18 erstreckt sich dann von der Befeuchtungseinrichtung 6 zur Nasenbrille 38.

Die Befeuchtungseinrichtung 6 ist hier mit einem Reservoir 16 für Wasser ausgestattet. Das Wasser wird verdampft und/oder vernebelt und mit der durch den Beatmungsschlauch 18 strömenden Atemluft vermischt. Zugleich wird die durch die Befeuchtungseinrichtung 6 strömende Atemluft über die Heizeinrichtung 7 gezielt aufgeheizt.

Für eine optimale Einstellung von Feuchte und/oder Temperatur der Atemgasströmung ist die Schlaucheinrichtung 8 hier mit einer Sensoreinheit 48 ausgestattet. Über diese Sensoreinheit 48 wird dann die Leistung der Befeuchtungseinrichtung 6 bzw. Heizeinrichtung 7 entsprechend geregelt.

Zusätzlich oder alternativ zu der Heizeinrichtung 7 kann die Schlaucheinrichtung 8 hier als Heizschlauch ausgebildet sein. Dazu weist der Beatmungsschlauch 18 eine eigene Heizung 58 auf. Dabei ist die Heizung 58 von dem Beatmungsschlauch 18 zerstörungsfrei trennbar, sodass die Heizung 58 nach dem Schlauchwechsel mit einem neuen Beatmungsschlauch 18 weiterverwendet werden kann. Die Heizung 58 kann über die Sensoreinheit 48 regelbar sein.

Das hier gezeigte System 1 ist mit einer Sensoreinrichtung 5 ausgestattet, welche hier nicht sichtbar in dem Beatmungsgerät 11 untergebracht ist. Über diese Sensoreinrichtung 5 erfasst die Steuereinrichtung 4 den Druck, den die Beatmungseinrichtung 2 zur Aufrechterhaltung des definierten Atemgasflusses aufbringen muss, um die Atemtätigkeit des Patienten auszugleichen. Beispielsweise wird dazu der Druck am Geräteausgang gemessen, welchen die Beatmungseinrichtung 2 benötigt hat, um den definierten Atemgasfluss konstant zu halten. Dieser Druck ändert sich laufend. Beispielsweise wird bei der Einatmung weniger Druck benötigt, um den definierten Atemgasfluss aufrechtzuerhalten, als bei der Ausatmung. Bei der Ausatmung wird hingegen mehr Druck benötigt, da der Patient gegen den applizierten Atemgasfluss atmet. Die Sensoreinrichtung 5 umfasst beispielsweise wenigstens einen Drucksensor und oder wenigstens einen Flusssensor.

Die Steuereinrichtung 4 registriert den Verlauf des erfassten Drucks über die Zeit und speichert diesen als Druckverlauf ab. Durch diesen Druckverlauf kann die Steuereinrichtung 4 hier eine für die Atemtätigkeit des Patienten charakteristische Kenngröße bestimmen. Beispielsweise bildet die Steuereinrichtung über den Druckverlauf das Atemmuster ab. Aus dem Atemmuster heraus können dann diverse Atemereignisse und Situationen erkannt werden. Darauf kann wiederum reagiert werden, beispielsweise mit entsprechenden Einstellungen der Beatmungseinrichtung und insbesondere Flowänderungen. Zudem können die Atemereignisse bzw. Atemmuster per Telemonitoring übertragen werden. Beispielsweise kann daraus der AHI ermittelt werden.

Die Steuereinheit 4 ist hier zur Erkennung von Atmungsbemühungen aus dem Drucksignal und/oder aus dem Flusssignal der Drucksensoreinrichtung 5 und/oder der Flusssensoreinrichtung 5 eingerichtet und ausgebildet. Die Steuereinheit 4 ist zudem zur Erkennung von periodischer Atmung oder Atemaussetzern aus dem Drucksignal und/oder aus dem Flusssignal der Drucksensoreinrichtung 5 und/oder der Flusssensoreinrichtung 5 eingerichtet und ausgebildet.

Das System 1 ist beispielsweise auch ausgebildet und eingerichtet, dass eine Sauerstoffbeimischung, über eine hier nicht näher dargestellte interne oder externe Sauerstoffquelle, bis 300 l/min oder mehr erfolgt.

Das System 1 ist beispielsweise ausgebildet und eingerichtet, dass die Steuereinheit 4 unter Berücksichtigung der Signale der Fluss- und/oder Drucksensoreinrichtung 5, eine Sauerstoffquelle zur Sauerstoffbeimischung aktiviert, die atemphasenabhängig moduliert wird. Beispielsweise ist daran gedacht, die Sauerstoffbeimischung so zu steuern, dass in Phasen der Inspiration dem Patienten mehr Sauerstoff zur Verfügung steht als zur Exspiration.

Das System 1 ist beispielsweise ausgebildet und eingerichtet, dass ein Pulsoximeter oder ein Co2-Messgerät adaptiert werden kann, welches dann mit dem System 1 kommuniziert und/oder von ihm mit Energie versorgt wird. Über die Messwerte dieser Geräte kann der Anwender - oder ein automatischer Algorithmus - die richtige Flussrate und die richtige Sauerstoff-Beimischung einstellen. Beispielsweise berücksichtigt die Steuereinheit Messwerte des Pulsoximeters und/oder des Co2-Messgerätes bei der Aktivierung / Steuerung der Sauerstoffquelle und/oder bei der Aktivierung / Steuerung des Flusssteuermoduls.

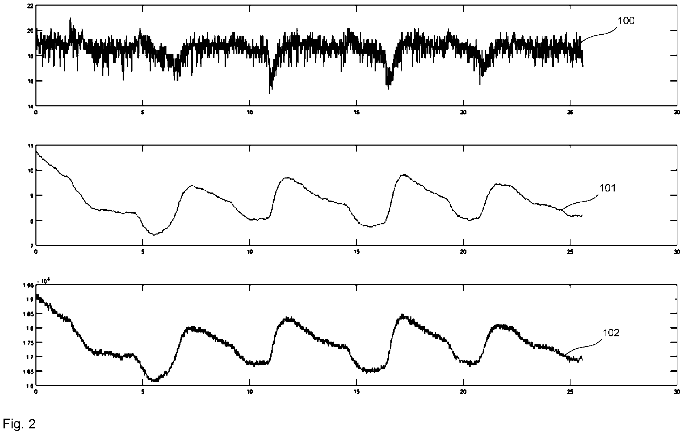

Die Figur 2 einen Flussverlauf 100 und einen Druckverlauf 101 sowie einen Drehzahlverlauf 102 der Drehzahl der Lüftereinrichtung 13, welche während einer beispielhaften Atemtätigkeit bzw. eines beispielhaften Betriebs des Systems 1 aufgezeichnet wurden. Dabei zeigt das obere Diagramm den Flussverlauf 100, bei dem die Flussrate in Liter pro Minute gegen die Zeit aufgetragen wurde. Das mittlere Diagramm zeigt den Druckverlauf 101, bei dem der von der Sensoreinrichtung 5 erfasste Druck über die Zeit aufgetragen wurde. Das untere Diagramm zeigt den Drehzahlverlauf 102, bei dem die Drehzahl in Umdrehungen pro Minute der Lüftereinrichtung 13 gegen die Zeit aufgetragen wurde. Die Werte wurden hier während einer Mundatmung des Patienten erfasst.

Der eingestellte Fluss ist hierbei 20 1 pro Minute. Aus der Aufzeichnung ist zu erkennen, dass der Fluss im Bereich 16 bis 20 l/min schwankt. Diese Schwankungen sind durch die Atemtätigkeit des Patienten zu erklären. Bei der Einatmung nimmt der Fluss zu. Mit Beginn der Ausatmung nimmt er wieder ab. Dies ist zu erklären durch den Staudruck, den der Patient mit seiner Ausatmung. Der Ausatmung wird der Fluss aus der Nasenbrille gering unterdrückt. Mit Beginn der Inspiration kann das Atemgas ungehindert aus der Patientenschnittstelle strömen.

Man erkennt, dass der Druck zwischen 7,5 und 10 mbar schwankt. Diese Schwankungen sind durch die Atmung des Patienten bedingt. Mit der Einatmung kann das Atemgas leichter aus der Patientenschnittstelle strömen, entsprechend nimmt der Druck im Schlauchsystem ab. Mit der Ausatmung steht dem Fluss des Atemgases der Fluss der ausgeatmeten Luft entgegen. Da die Steuereinheit 4 versucht, den Fluss konstant zu halten, muss sie während der Ausatmung den Druck erhöhen, um dieselbe Flussleistung aus der Patientenschnittstelle Strömen zu lassen.

Man erkennt, dass die Drehzahl in einem bestimmten Bereich schwankt. Die Schwankungen korrelieren mit den Schwankungen im Signal des Druckverlaufs 101. Die Schwankungen entsprechen auch den Schwankungen im Signal des Flussverlaufs 100. Damit der Fluss während der Einatmung konstant gehalten wird, muss die Steuereinheit 4 die Drehzahl leicht absenken. Während der Ausatmung versucht die Steuereinheit 4, den Fluss auf einem vorgegebenen Niveau zu halten. Dafür ist die Drehzahl anzuheben, um den Druck zu erhöhen und um über den erhöhten Druck einen gleichbleibenden Fluss zu gewährleisten.

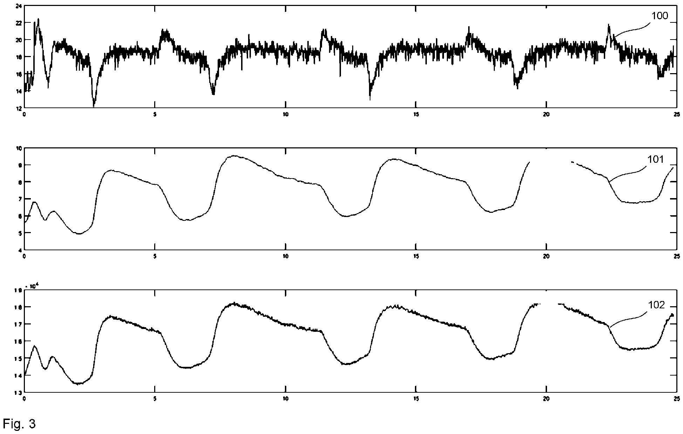

Die Figur 3 zeigt analog zu der Figur 2 im oberen Diagramm den Flussverlauf 100 und im mittleren Diagramm den Druckverlauf 101 und im unteren Diagramm den Drehzahlverlauf 102. Im Gegensatz zu der Figur 2 wurden die Werte hier bei einer an Nasenatmung erfasst. Dabei war der Mund geschlossen und der Patient atmete durch die Nase.

Für die Erkennung des AHI als Kenngröße wird beispielsweise eine Abnahme der benötigten Druck-Schwankung zur Ausregelung des Atemgasflusses um x% herangezogen. Die Abnahme der Druck-Schwankung X liegt dabei bevorzugt zwischen 30 % und 50% bei Hypopnoen und/oder zwischen 70 % und 100% bei Apnoen. Alternativ oder ergänzend kann eine Zeitdauer für die Abnahme der Druck-Schwankung herangezogen werden; beispielsweise ist die Zeitdauer mindestens 5 Sekunden, bevorzugt mindestens 10 Sekunden. Die Abnahme der Druck-Schwankung muss demnach mindestens 5 Sekunden andauern, um eine Apnoe oder Hypopnoe zu erkennen. Es kann auch eine Zählung der Apnoen und Hypopnoen pro Stunde (AHI) vorgenommen werden. Insbesondere wird mindestens ein AHI-Wert pro Tag gespeichert. Zudem wird eine Unterscheidung in obstruktive und zentrale Apnoen und Hypopnoen vorgenommen. Die Ausregelung des permanenten Atemgasflusses über den Druck kann näherungsweise erfolgen. Ein bevorzugtes Ziel ist es dabei, dass der Atemgasfluss um weniger als 5 l/min und/oder um weniger 10% schwankt. Wenn die Ausregelung nur außerhalb eines Toleranzfeldes gelingt, kann alternativ zur Ermittlung der Atemtätigkeit der Druckschwankung und Atemgasfluss-Schwankung gewichtet addiert werden. Bei Erkennung einer dauerhaften Apnoe und/oder Hypopnoe kann vorgesehen sein, dass das Gerät darüber ein nicht korrekt angelegtes Patienteninterface erkennt und dann z. B. einen Alarm ausgibt. Zum Beispiel kann "Patienten-Interface verrutscht" ausgegeben werden. Des Weiteren kann eine Erkennung von intermittierender erhöhter Atmungstätigkeit als Hustenstöße vorgesehen sein. Zum Beispiel erfolgt eine Zählung der Hustenstöße pro Stunde und Speicherung mindestens eines Wertes pro Tag.

In einer vorteilhaften Ausgestaltung ist (insbesondere als Kenngröße und/oder mittels der Kenngröße) eine DekompensationsErkennung vorgesehen. Darunter wird insbesondere eine Erkennung des Verdachts einer Exazerbation verstanden. Die Erkennung erfolgt Z. B. über einen Anstieg der Atemfrequenz, insb. auf über 20 bpm oder um 20%, und/oder über einen Abfall der basalen Sauerstoff-Sättigung, insb. auf unter 90 % oder 85% oder um 5ö, und/oder über einen Anstieg der Pulsfrequenz und/oder über einen Anstieg der Anzahl der Hustenstöße pro Stunde oder pro Tag und/oder über einen Anstieg der Anzahl Apnoen pro Stunde oder pro Tag und/oder über einen Anstieg oder Abfall der Nutzungsdauer.

In einer ebenfalls vorteilhaften Ausgestaltung ist eine Monitoring-Schnittstelle vorgesehen. Diese kann für ein Monitoring im Krankenhaus / Pflegeheim ausgelegt sein und z. B. Bluetooth, PDMS-Schnittstelle, LAN, WLAN umfassen. Die Schnittstelle kann für ein Offline-Monitoring zu Hause ausgelegt sein und z. B. einen internen Gerätespeicher, Speichermedium, Kommunikation über Bluetooth mit einer App umfassen. Die Schnittstelle kann auch für ein Tele-Monitoring ausgelegt sein und z. B. als IoT Schnittstelle ausgebildet sein und beispielsweise GSM, NB-IoT, LTE-M, 2G, 4G, 5G umfassen bzw. dazu geeignet sein. Die Schnittstelle kann Signale übertragen, insbesondere Atemgasdruck und Atemgasfluss. Die Schnittstelle kann gespeicherte Statistik übertragen und insbesondere Mittelwerte und/oder Mediane und/oder Perzentile und/oder Histogramme, welche sich vorzugsweise auf Atemfrequenz, Druckschwankungen, Atemgasfluss-Schwankungen, SpO2-Werte, Pulsfrequenz-Werte, FiO2-Werte beziehen bzw. diese beschreiben. Die Schnittstelle kann gespeicherte Ereignisse übertragen und insbesondere Anzahl Apnoen und Hypopnoen pro Stunde pro Tag oder pro Tag, Anzahl Sauerstoff-Entsättigungen pro Stunde pro Tag oder pro Tag, Anzahl Hustenstöße pro Stunde pro Tag oder pro Tag. Die Schnittstelle kann kann Alarme übertragen und z. B. Interface-Alarme, Frequenz-Alarme und/oder Dekompensations-Alarme. Die Schnittstelle kann Nutzungsdauern und/oder Nutzungszeiten insbesondere samt gewählter Einstellparameter übertragen. Insbesondere können Einstellparameter und/oder Geräteparameter und/oder Beatmungsparameter über die Schnittstelle verändert werden. Vorzugsweise kann sich das Gerät authentifizieren, bevorzugt mit seiner Seriennummer und/oder einem Sicherheitsschlüssel, z. B. einem Zertifikat.

Bei einer Atemphasen-Erkennung als Kenngröße erfolgt mittels der Kenngröße vorzugsweise wenigstens eine Messung der Atemfrequenz insbesondere über die Formel: 60 / Dauer eines Atemzuges (in s). Insbesondere erfolgt in Abhängigkeit der Atemphase als Kenngröße eine Steuerung der Sauerstoff-Beimischung (inspiratorisch mehr als exspiratorisch). Insbesondere erfolgt in Abhängigkeit der Atemphase als Kenngröße eine Steuerung der Atemgas-Befeuchtung und - erwärmung (inspiratorisch mehr als exspiratorisch). Insbesondere erfolgt in Abhängigkeit der Atemphase als Kenngröße eine Steuerung des Atemgasflusses: inspiratorisch mehr als exspiratorisch (Bilevel-Highflow).

### Bezugszeichenliste:

- 1: System
- 2: Beatmungseinrichtung
- 3: Atemgasquelle
- 4: Steuereinrichtung
- 5: Sensoreinrichtung
- 6: Befeuchtungseinrichtung
- 7: Heizeinrichtung
- 8: Schlaucheinrichtung
- 10: Bedieneinrichtung
- 11: Beatmungsgerät
- 13: Lüftereinrichtung
- 16: Reservoir
- 18: Beatmungsschlauch
- 20: Anzeigeeinrichtung
- 28: Patientenschnittstelle
- 38: Nasenbrille
- 48: Sensoreinheit
- 58: Heizung
- 100: Flussverlauf
- 101: Druckverlauf
- 102: Drehzahlverlauf

## Patentansprüche

1. System (1) zur Atemgasversorgung mit wenigstens einer Beatmungseinrichtung (2) mit wenigstens einer Atemgasquelle (3) zur Erzeugung einer Atemgasströmung und mit wenigstens einer Steuereinrichtung (4), wobei die Steuereinrichtung (4) dazu geeignet und ausgebildet ist, mittels der Beatmungseinrichtung (2) wenigstens einen definierten Atemgasfluss für eine Beatmung zu erzeugen, **dadurch gekennzeichnet, dass** die Steuereinrichtung (4) dazu geeignet und ausgebildet ist, mittels wenigstens einer Sensoreinrichtung (5) wenigstens einen für einen Druck charakteristischen Parameter zu überwachen und dass der Parameter denjenigen Druck charakterisiert, mit welchem die Beatmungseinrichtung (2) die Atemgasströmung zur Aufrechterhaltung des definierten Atemgasflusses beaufschlagt, um eine Atemtätigkeit des Patienten auszugleichen und dass die Steuereinrichtung (4) dazu geeignet und ausgebildet ist, einen Verlauf des Parameters über die Zeit als einen Druckverlauf zu registrieren und anhand des Druckverlaufs wenigstens eine für die Atemtätigkeit des Patienten charakteristische Kenngröße zu bestimmen, wobei das System dazu geeignet und ausgebildet ist, bestimmungsgemäß mit einer Atemmaske als Patientenschnittstelle (28) betrieben zu werden, wobei die Atemmaske dazu geeignet und ausgebildet ist zumindest die Nase des Patienten zu umschließen und einen Leckagestrom im Wesentlichen zu verhindern, wobei die Steuereinrichtung (4) dazu geeignet und ausgebildet ist, die Lüftereinrichtung so zu betreiben, dass die einen, der Atemtätigkeit des Patienten entgegenwirkenden, im Wesentlichen gleichbleibenden Fluss erzeugt.

2. System (1) nach einem der vorhergehenden Ansprüche, wobei die Steuereinrichtung (4) dazu geeignet und ausgebildet ist, eine Einatmung oder Ausatmung dadurch zu erkennen, dass der zur Aufrechterhaltung des definierten Atemgasflusses benötigte Druck über die Zeit abnimmt oder zunimmt.

3. System (1) nach einem der vorhergehenden Ansprüche, wobei die Steuereinrichtung (4) dazu geeignet und ausgebildet ist, anhand des Druckverlaufs wenigstens ein Atemmuster oder ein Atemereignis zu bestimmen und das Atemmuster auf wenigstens ein Atemereignis zu untersuchen.

4. System (1) nach einem der vorhergehenden Ansprüche, wobei die Steuereinrichtung (4) dazu geeignet und ausgebildet ist, abhängig vom Druckverlauf wenigstens eine Benutzerinteraktion, beispielsweise einen Alarm, auszugeben und/oder den Druckverlauf, insbesondere ein darüber erkanntes Atemereignis und/oder Atemmuster, an eine Netzwerkeinrichtung zu übertragen.

5. System (1) nach einem der vorhergehenden Ansprüche, wobei die Steuereinrichtung (4) dazu geeignet und ausgebildet ist, in Abhängigkeit des Druckverlaufs wenigstens einen Geräteparameter der Beatmungseinrichtung (2) einzustellen und/oder mittels der Beatmungseinrichtung (2) wenigstens einen Beatmungsparameter einzustellen, wobei die Einstellung des Geräteparameters und/oder Beatmungsparameters, vorzugsweise einer Flussrate des Atemgasflusses, in Abhängigkeit eines erkannten Atemmusters und/oder Atemereignisses erfolgt.

6. System (1) nach einem der vorhergehenden Ansprüche, wobei die Steuereinrichtung (4) dazu geeignet und ausgebildet ist, mittels der Beatmungseinrichtung (2) wenigstens eine Flussrate des Atemgasflusses in Abhängigkeit einer Sättigung des Atemgasflusses mit Sauerstoff einzustellen, wobei die Steuereinrichtung (4) dazu geeignet und ausgebildet ist, in Abhängigkeit des Druckverlaufs eine Sauerstoffbeimischung in die Atemgasströmung einzustellen und insbesondere die Sauerstoffbeimischung atemphasenabhängig zu modulieren, wobei die Sauerstoffbeimischung und/oder ein Sauerstoffgehalt im Atemgasfluss um ein bestimmtes Maß verringert wird, wenn eine Flussrate des Atemgasflusses erhöht wird.

7. System (1) nach einem der drei vorhergehenden Ansprüche, wobei die Sauerstoffbeimischung nur während einer Einatmung und/oder nicht während einer Ausatmung erfolgt und/oder wobei die Sauerstoffbeimischung während einer Einatmung gegenüber einer Ausatmung erhöht wird.

8. System (1) nach einem der vorhergehenden Ansprüche oder nach dem Oberbegriff von Anspruch 1, umfassend wenigstens eine Befeuchtungseinrichtung (6) zur Befeuchtung der Atemgasströmung und/oder wenigstens eine Heizeinrichtung (7) zur Beheizung der Atemgasströmung, wobei die Steuereinrichtung (4) dazu geeignet und ausgebildet ist, in Abhängigkeit des Druckverlaufs eine Befeuchtung und/oder Beheizung der Atemgasströmung einzustellen und insbesondere auf eine Zieltemperatur und/oder Zielfeuchte des Atemgasflusses zu regeln, wobei die Heizeinrichtung (7) dazu geeignet und ausgebildet ist die Atemgasströmung (zumindest in der Inspiration) auf 36 - 38 °C zu erwärmen und wobei die Befeuchtungseinrichtung (6) dazu geeignet und ausgebildet ist die Atemgasströmung mit einer relativen Feuchtigkeit im Bereich von 90 - 100%, bevorzugt 95 - 99%, zu befeuchten, wobei im Nachlauf einer Beatmung wenigstens ein Trocknungsmodus durchführbar ist.

9. System (1) nach einem der beiden vorhergehenden Ansprüche, wobei mittels der Steuereinrichtung (4) eine atemphasengesteuerte Befeuchtung und/oder Beheizung einstellbar ist, wobei die Steuereinrichtung (4) dazu geeignet und ausgebildet ist, die Befeuchtung und/oder Beheizung in Abhängigkeit einer Flussrate des Atemgasflusses einzustellen und/oder wobei die Steuereinrichtung (4) dazu geeignet und ausgebildet ist, die Befeuchtung und/oder Beheizung in Abhängigkeit wenigstens einer sensorisch erfassten Größe einzustellen und wobei die Größe vorzugsweise aus einer Gruppe von Größen entnommen ist, umfassend wenigstens: Umgebungstemperatur, Umgebungsfeuchte, Temperatur im System, Feuchte im System (1), Schlauchtemperatur, Temperatur an einer Patientenschnittstelle, Feuchte am Schlauchende und/oder an einer Patientenschnittstelle (28).

10. System (1) nach einem der fünf vorhergehenden Ansprüche, wobei die Steuereinrichtung (4) dazu geeignet und ausgebildet ist, durch die Befeuchtung und/oder Beheizung verursachte Flowfehler und/oder andere Abweichungen mittels der Beatmungseinrichtung wenigstens näherungsweise zu kompensieren.

11. System (1) nach einem der sechs vorhergehenden Ansprüche, wobei die Steuereinrichtung (4) dazu geeignet und ausgebildet ist, die für die Beatmung vorgesehene Flussrate des Atemgasflusses solange zu reduzieren, bis ein Maß für die Feuchte des Atemgasflusses einen Schwellenwert erreicht und/oder bis die Temperatur des Atemgasflusses einen Schwellenwert erreicht.

12. System (1) nach einem der vorhergehenden Ansprüche oder nach dem Oberbegriff von Anspruch 1, umfassend wenigstens eine Schlaucheinrichtung (8) mit wenigstens einem an die Beatmungseinrichtung (2) koppelbaren Beatmungsschlauch (18) zum Zuführen der Atemgasströmung an eine Patientenschnittstelle (28) und/oder wobei die Schlaucheinrichtung (8) wenigstens einen beheizbaren Beatmungsschlauch (18) umfasst und wobei der beheizbare Beatmungsschlauch (18) wenigstens einen Teil einer Sensoreinheit (48) zur Erfassung wenigstens einer für die Feuchte und/oder Temperatur charakteristischen Größe umfasst, wobei die Schlaucheinrichtung (8) wenigstens einen graduell über seine Länge beheizbaren Beatmungsschlauch (18) umfasst, wobei die Schlaucheinrichtung (8) wenigstens einen beheizbaren Beatmungsschlauch (18) mit wenigstens einer von dem Beatmungsschlauch (18) trennbaren Heizung (58) umfasst, wobei die Heizung (58) bei einem Schlauchwechsel mit einem neuen Beatmungsschlauch (18) bestimmungsgemäß ausrüstbar und weiterverwendbar ist oder wobei die Schlaucheinrichtung (8) wenigstens einen Beatmungsschlauch (18) aus einem Wasser ausleitenden Material aufweist und/oder wenigstens einen wärmeisolierten Beatmungsschlauch (18) aufweist.

13. System (1) nach einem der vorhergehenden Ansprüche, wobei die Steuereinrichtung (4) dazu geeignet und ausgebildet ist, mittels der Beatmungseinrichtung (2) durch den definierten Atemgasfluss innerhalb einer vorgegebenen Inspirationszeit ein festgelegtes Atemvolumen zu applizieren und wobei die Steuereinrichtung (4) dazu geeignet und ausgebildet ist, für den definierten Atemgasfluss wenigstens einen hinterlegten Maximaldruck zu berücksichtigen.

14. System (1) nach einem der vorhergehenden Ansprüche, wobei der definierte Atemgasfluss eine Flussrate im Bereich von 0
- 110 l/min, bevorzugt 1 - 80 l/min, besonders bevorzugt 2
- 60 l/min aufweist oder wobei der definierte Atemgasfluss eine Flussrate für Säuglinge, per auswählbarer Voreinstellung, im Bereich von 1 - 10 l/min aufweist.

15. System (1) nach einem der vorhergehenden Ansprüche, dazu geeignet und ausgebildet ist, bestimmungsgemäß mit einer Nasenbrille (38) als Patientenschnittstelle (28) betrieben zu werden, wobei die Nasenbrille (38) für jedes Nasenloch einen Stutzen (38a, 38b) aufweist, wobei jeder Stutzen zumindest teilweise in das jeweilige Nasenloch einführbar ist und wobei jeder Stutzen einen Durchmesser aufweist, der so bemessen ist, dass das jeweilige Nasenloch bei eingeführtem Stutzen nicht dicht verschlossen ist, wobei jeder Stutzen (38a, 38b) einen Durchmesser aufweist, der weniger als 9/10, bevorzugt weniger als 8/10 und mehr als 6/10, des Durchmessers des jeweiligen Nasenloches beträgt.
